(19) Europäisches Patentamt
European Patent Office
Office européen des brevets

(11) Publication number: **0 129 031**
**A2**

# (12) EUROPEAN PATENT APPLICATION

(21) Application number: 84105031.3

(22) Date of filing: 04.05.84

(51) Int. Cl.$^3$: **A 61 K 31/70**
A 61 K 31/645, A 61 K 31/195

(30) Priority: 19.05.83 JP 87968/83
19.05.83 JP 87969/83
19.05.83 JP 87970/83

(43) Date of publication of application:
27.12.84 Bulletin 84/52

(84) Designated Contracting States:
AT BE CH DE FR GB IT LI LU NL SE

(71) Applicant: Suntory Limited
1-40 Dojimahama 2-chome Kita-ku
Osaka-shi Osaka-fu 530(JP)

(72) Inventor: Suwa, Yoshihide
7-16, Yamatedai 6-chome
Ibaraki-shi Osaka(JP)

(72) Inventor: Kobayashi, Takumi
1-10, Himuro-cho 1-chome
Takatsuki-shi Osaka(JP)

(72) Inventor: Kiyota, Noriko
14-4, Aoyamadai 4-chome
Suita-shi Osaka(JP)

(72) Inventor: Yoshizumi, Hajime
6-1-612, Kosobe-cho 2-chome
Takatsuki-shi Osaka(JP)

(74) Representative: Hansen, Bernd, Dr.rer.nat. et al,
Hoffmann, Eitle & Partner Patentanwälte Arabellastrasse
4
D-8000 München 81(DE)

## (54) Antimutagenic agent.

(57) Coenzyme A, pyridoxal-5'-phosphoric acid, as well as pantothenic acid and alkali metal or alkaline earth metal salts thereof are active as antimutagenic agents. Mutagenicity if believed to be responsible for most causes of human cancer, and there are certain foods that have proved to contain mutagens. However, their mutagenicity can be sufficiently suppressed by ingesting them together with the compounds listed above. These compounds are either vitamins or their endoforms, and hence can be safely incorporated in foods.

EP 0 129 031 A2

Croydon Printing Company Ltd

# ANTIMUTAGENIC AGENT

The present invention relates to an antimutagenic agent having as its effective ingredient a compound selected from the group consisting of coenzyme A, pyridoxal-5'-phosphoric acid, as well as pantothenic acid and alkali metal or alkaline earth metal salts thereof.

Most of the cancers that attack humans are said to originate from carcinogenic factors present in their living environment. Only about 2% of the human cancers can be explained on a hereditary basis (Higginson, J. & Muir, C.S.: J. Natl. Cancer Inst., Vol. 63, 1291, 1979). Cancers now account for about one fourth to one fifth of the causes of human death, and there is a great accumulation of epidemiological data that supports the hypothesis that the foods and beverages we ingest every day may be the arch-criminal of human cancers. Carcinogens originating from foods and beverages are classified into two types; one which is already present in foods and beverages when they are ingested by humans, and the other which is formed in the digestive tract as a result of nitrosation. Carcinogens of the first type are polycyclic aromatic hydrocarbon compounds such as benzo[a]pyrene, benz[a]anthracene, benzo[b]chrysene, phenanthrene and dibenz[a,h]anthracene, and nitroso compounds such as dimethylnitrosoamine, nitrosopyrrolidine and N'-nitrosonornicotine. These carcinogens are detected in tobacco tar, sirloin steak, pork chop, barbecue, grilled fish, salami and fried bacon. The second type of carcinogens is how becoming the subject of researchers' attention as another cause of stomach cancer. When a certain foodstuff is ingested, nitrous acid in the saliva nitrosates secondary amines or amide under acidic conditions (in the gastric juice). The nitrosation of a secondary amine may be represented by the following reaction scheme:

$$2HNO_2 \rightleftharpoons N_2O_3 + H_2O$$

$$R_2NH + N_2O_3 \longrightarrow R_2N{\cdot}NO + HNO_2$$

The rate for this reaction is given by $K_1[R_2NH][HNO_2]^2$ wherein $K_1$ is a constant for the reaction rate.

Nitrous acid is also contained in vegetables that have lost some of their freshness (e.g. lightly salted vegetable) or is incorporated in fish sausage of ham as an additive. Soybean sauce, Banbanchī source and horsebeans cause a high level of mutagenicity when they are nitrosated. In other words, they contain mutagen precursors. It has been shown epidemiologically that the intake level of nitrous acid and the mutagen precursor containing products has high correlation with the incidence of stomach cancer (Correa. P. et al.: Lancet, ii, 58, 1975; Tannenbaum, S. R. et al.: J. Natl. Cancer Inst., Vol. 62, 9, 1979). Despite intensive efforts made by researchers on the structures of nitroso compounds formed in the gastric juice, few substances have been identified for their chemical structure. However, researchers at the National Cancer Center who had been conducting experiments on the mutagenicity that was closely corrected to carcinogenicity found in 1982 that the precursor of a nitroso compound that was produced as a result of reaction between soybean sauce and nitrous acid under acidic conditions (pH 3.0) was 1,2,3,4-tetrahydroharman-3-carboxylic acid (Wakabayashi et al., Proc. of Meeting of The Environmental Mutagen Socieity of Japan, p. 76, 1982).

In the absence of a sovereign medicine against cancers today, it is strongly desired to eliminate carcinogenic factors from the living environment or to inactivate them completely. Therefore, many efforts are being directed to the elimination or inactivation of carcinogens (or mutagens). Peroxidase, thiol compounds and selenium compounds have been reported to have the ability to inactivate the mutagenicity of Trp-P-1, N-methyl-N'-nitro-N-nitrosoguanidine and 7,12-dimethylbenzanthracene, respectively (Martin, s.e. et al.: Mutation Res., Vol. 82, 41, 1981). However, many of the inactivators known today are either toxic to the living body or unsuitable for daily intake due to their poor palatability. Vitamin C is able

to inhibit nitrosation and can be safely ingested by human as an essential nutrient. However, vitamin C is unable to inactivate a nitroso compound already produced in the human body. The development of an inactivator or cancer preventive that may be safely ingested by humans and which exhibits its activity against a broad spectrum of carcinogens in the living environment is urgently needed.

One of the methods for determining carcinogenicity that have recently been established is the Salmonella test (also known as the Ames test) that uses the requirement for histidine as an evaluation index (Ames, B.N. et al.: Mutation Res., Vol. 31, 347, 1975). Many research institutes checked the mutagenicity of compounds by the Ames method and have confirmed that the correlation of the result with their carcinogenicity is an high as 80 to 90% (McCann. J. et al.: Proc. Natl. Acad. Sci. (U.S.A.), Vol. 72, 5153, 1975). Therefore, the present inventors chose to use the Ames test as a means for screening various compounds to discover cancer control substances.

Substances that can be ingested by humans for inactivating the mutagenicity of carcinogens should be such that the absence of their toxicity to the living body has already been confirmed. With this in mind, the present inventors have made various efforts to find the desired inactivator and have finally identified coenzyme A, pyridoxal-5'-phsophoric acid and pantothenic acid as antimutagenic substances.

Coenzyme A inhibits completely the mutagenicity of N-methyl-N'-nitro-N-nitrosoguanidine (MNNG), a substance to cause cancer of the stomach or intestines, if 150 mols of coenzyme A is added to 1 mol of MNNG. This coenzyme is also capable of inactivating about 90% of the mutagenicity of benzo[a]pyrene,a substance known to be a potent carcinogen to the liver and skin, by adding 10 mols of the coenzyme to 1 mol of this carcinogen.

Pyridoxal-5'-phosphoric acid inhibits about 50% of the mutagenicity of MNNG by adding 150 mols of this compound

to 1 mol of MNNG. The mutagenicity of benzo[a]pyrene can be inactivated by about 80% if 25 mols of the compound is added to 1 mol of the carcinogen.

Nitrosoamines are present in sausage, bacon, mushrooms, and cod in concentrations of 0.8-2.4 ppb, 0.6-6.5 ppb, 1.4-30 ppb, and 15 ppb respectively. Dimethylnitrosoamine is present in a cigarette in a concentration of 1-200 ng, and nitrosopyrrolidine in a concentration of 2-42 ng. Benzo[a]pyrene is contained in coffee, black tea, sausage, steak and a cigarette in concentrations of 0.3-1.3 ppb, 3.9 ppb, 12.5-18.8 ppb, 8.0 ppb, and 1-50 ng, respectively. In theory based on the calculated molar ratio necessary for inactivating these carcinogens, their mutagenicity can be satisfactorily eliminated by ingesting either coenzyme A or pyridoxal-5'-phosphoric acid in doses of the order of milligrams.

As another advantages coenzyme A and pyridoxal-5'-phosphoric acid will present no potential hazard to human since, as shown below, they are respectively endo-forms of pantothenic acid and vitamin $B_6$:

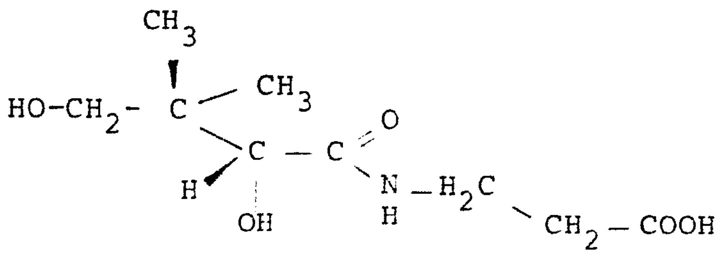

pantothenic acid

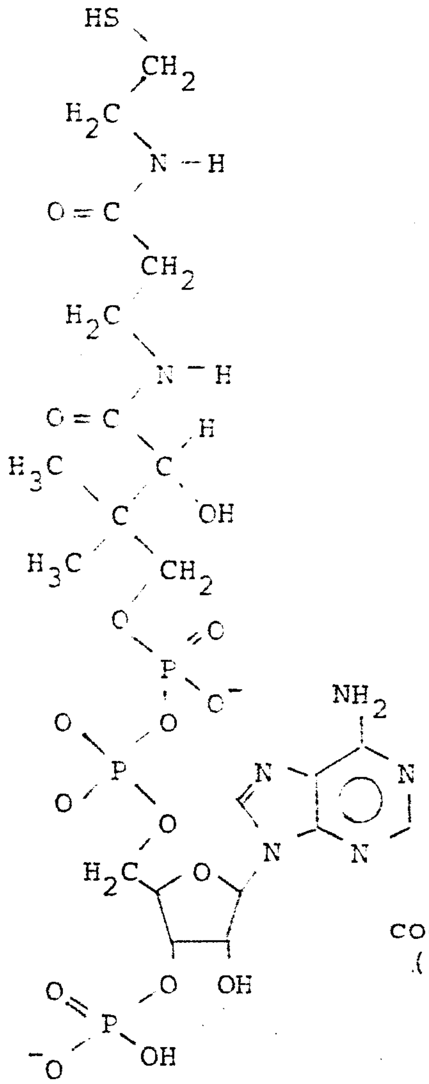

coenzyme A
(in vivo)

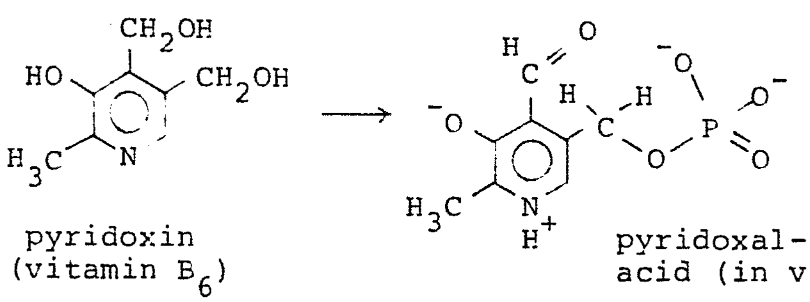

pyridoxin
(vitamin $B_6$)

pyridoxal-5'-phosphoric
acid (in vivo)

Furthermore, the vitamin activities of coenzyme A and pyridoxal-5'-phosphoric acid, namely, their therapeutic effects against vitamin deficiencies, are comparable to those of pantothenic acid and vitamin $B_6$. Therefore, these compounds when ingested by the human body will work not only as antimutagens but also as vitamins.

Pantothenic acid suppresses the mutagenicity of MNNG and 1-methyl-1-nitrosourea (MNU), another N-nitroso compound that causes cancer in the stomach and intestines. Pantothenic acid also suppresses the mutagenicity of 4-nitrosoquinoline N-oxide (4NQO) and 2[(2-furyl)-3-(5-nitro-2-furyl)]acrylamide (AF-2), both of which are heterocyclic compounds that induce cancer of the stomach. Other substances the mutagenicity of which can be suppressed by pantothenic acid are adriamycin which is both a cancer control agent and a carcinogen, and methylglyoxal which is contained in coffee and is known to be a mutagen although its carcinogenicity is yet to be confirmed.

The above listed N-nitroso compounds are present in a cigarette, fish (e.g. smoked salmon, mackerel or herring), mushrooms, hamburger and bacon or ham in concentrations of 0-180 ng, 0.5-40 ppb, 0.4-30 ppb, 15-25 ppb and 0.6-6.5 ppb, respectively. Again, in theory based on the calculated molar amount necessary for inactivating these mutagens, their mutagenicity can be sufficiently eliminated by ingesting pantothenic acid in doses of the order of milligrams. Pantothenic acid is a compound classified as vitamin B and hence is entirely safe to the human body. Furthermore, pantothenic acid has its own physiological activities (metabolism of lipids, energy and amino acids) which are beneficial to the man who has ingested the acid.

The ability of coenzyme A, pyridoxal-5'-phosphoric acid and pantothenic acid to inactivate the mutagenicity of carcinogens and mutagens is entirely a novel and interesting finding and will play an important roll in the future efforts toward cancer prevention.

Pantothenic acid exhibits the antimutagenic effect even if it is in the form of an alkali metal or alkaline

earth metal. Pantothenyl alcohol of formula I also works as an antimutagenic substance by being converted to pantothenic acid as soon as it is ingested by the human body:

$$\begin{array}{c} CH_2OH \\ | \\ CH_2 \\ | \\ CH_2 \\ | \\ NH \\ | \\ CO \\ | \\ HCOH \\ | \\ CH_3-C-CH_3 \\ | \\ CH_2OH \end{array} \qquad I$$

The advantages of the present invention will become apparent by reading the following examples to which the scope of the invention is by no means limited.

Example 1

Efficacy of Coenzyme A

(I) Measuring mutagenicity and the antimutagenic effect

(i) Method: The preincubation method shown in Sugimura & Nagao: Chemical Mutagens, Vol. 6, page 41, 1981 was used.

(ii) Microorganism: Histidine requiring *Salmonella typhimurium* strains TA 100 and TA 98 (hereunder TA 100 and St TA 98) were used.

(iii) Preparation of samples

a) MNNG: To MNNG samples (1 μg/plate), coenzyme A was added in amounts of 0.01 μM, 0.1 M and 1.0 μM/plate.

b) Benzo[a]pyrene: To benzo[a]pyrene samples (10 μg/plate), coenzyme A was added in amounts of 0.0009, and 0.09 μM/plate.

(iv) Measuring mutagenicity

To 100 μl of each of the samples prepared in (iii) a), 500 μl of 100 mM sodium phosphate buffer (pH 7.4) and 100 μl of a culture of St TA 100 were added. To 100 μl of each of the samples prepared in (iii) b), 500 μl of S9 mix (rat liver homogenate) and 100 μl of a culture of TA 98 were added. The respective mixtures were shaken for 20 minutes at 37°C, and after adding 2.5 ml of a soft agar solution, the mixtures were spread on minimum glucose agar plates which had been supplemented with 50 μM/plate of

0129031

histidine for causing several meioses of the cells. After incubation at 37°C for 48 hours, the number of colonies on the plates was counted as revertants.

(II) Results

(i) Inactivation of the mutagenicity of MNNG by coenzyme A

Table 1

Efficacy of Coenzyme A against MNNG

| Coenzyme A (μM/plate) | No. of revertants/ plate | (%)* |
|---|---|---|
| 0 | 299 | 100 |
| 0.01 | 160 | 54 |
| 0.1 | 34 | 11 |
| 1.0 | 0 | 0 |

* The number of revertants per plate in the absence of coenzyme A was taken as 100%.

MNNG: 1 μg (6.8 nM)/plate

The effectiveness of coenzyme A against MNNG is clear from the data shown in Table 1.

(ii) Inactivation of the mutagenicity of benzo[a]pyrene by coenzyme A

Table 2

Efficacy of Coenzyme A against Benzo[a]pyrene

| Coenzyme A (μM/plate) | No. of revertants/ plate | (%)* |
|---|---|---|
| 0 | 461 | 100 |
| 0.0009 | 388 | 84 |
| 0.009 | 226 | 49 |
| 0.09 | 172 | 37 |
| 0.45 | 43 | 9 |

* The number of revertants per plate in the absence of coenzyme A was taken as 100%

Benzo[a]pyrene: 10 μg (39.6 nM)/plate

The effectiveness of coenzyme A against benzo[a]-pyrene is clear from the data shown in Table 2.

Example 2

Efficacy of Pyridoxal-5'-Phosphoric Acid

(I) Measuring mutagenicity and the antimutagenic effect

(i) Method: See Example 1.

(ii) Microorganism: See Example 1.

(iii) Preparation of samples

a) MNNG: To MNNG samples (1 μg/plate), pyridoxal-5'-phosphoric acid was added in amounts of 0.01, 0.1 and 1.0 M/plate.

b) Benzo[a]pyrene: To benzo[a]pyrene samples (10 μg/plate), pyridoxal-5'-phosphoric acid was added in amounts of 0.01, 0.1 and 1.0 μM/plate.

(iv) Measuring mutagenicity

See Example 1.

(II) Results

(i) Inactivation of the mutagenicity of MNNG by pyridoxal-5'-phosphoric acid

Table 3

Efficacy of Pyridoxal-5'-phosphoric acid against MNNG

| Pyridoxal-5'-phosphoric acid (μM/plate) | No. of revertants/ plate | (%)* |
|---|---|---|
| 0 | 299 | 100 |
| 0.01 | 297 | 99 |
| 0.1 | 249 | 83 |
| 1.0 | 152 | 51 |

* The number of revertants per plate in the absence of pyridoxal-5'-phosphoric acid was taken as 100%.

MNNG: 1 μg (6.8 nM)/plate

The effectiveness of pyridoxal-5'-phosphoric acid against MNNG is clear from the data shown in Table 3.

(ii) Inactivation of the mutagenicity of benzo[a]pyrene by pyridoxal-5'-phosphoric acid

Table 4

Efficacy of Pyridoxal-5'-phosphoric acid against Benzo[a]pyrene

| Pyridoxal-5'-phosphoric acid ( μM/plate) | No. of revertants/ plate | (%)* |
|---|---|---|
| 0 | 461 | 100 |
| 0.01 | 398 | 86 |
| 0.1 | 350 | 76 |
| 1.0 | 86 | 19 |

* The number of revertants per plate in the absence of pyridoxal-5'-phosphoric acid was taken as 100%

Benzo[a]pyrene: 10 μg (39.6 nM)/plate

The effectiveness of pyridoxal-5'-phosphoric acid against benzo[a]pyrene is clear from the data shown in Table 4.

Example 3

Efficacies of salts of Pantothenic Acid

(I) Measuring mutagenicity and the antimutagenic effect

(i) Method: See Example 1.

(ii) Microorganism: See Example 1.

(iii) Preparation of samples

a) The carcinogenic or mutagenic substances used in Example 3 and their concentrations are listed in Table 5.

Table 5

Carcinogenic or Mutagenic Substances and Their Concentrations

| Samples | Concentrations (μM/plate) |
|---|---|
| MNNG | 0.0068 |
| MNU | 0.85 |
| 4-NQO | 0.00066 |
| AF-2 | 0.00008 |
| Adriamycin | 0.0002155 |
| Methylglyoxal | 0.278 |

b) Adding pantothenates (Na and Ca salts)

Pantothenates were added to each of the carcinogenic or mutagenic samples in amounts of 0, 0.005, 0.05, and 5.0 μM/plate.

(iv) Measuring mutagenicity

To 100 μl of each of the samples prepared in (iii) a), 500 μl of 100 mM sodium phosphate buffer (pH 7.4) was added. Thereafter, 100 μl of a culture of St TA 100 or St TA 98 strain was added as indicated in Table 6.

Table 6

Culture of Strain Added

| Samples | Strain Culture |
|---|---|
| MNNG | S. TA 100 |
| MNU | S. TA 100 |
| 4NQO | S. TA 100 |
| AF-2 | S. TA 100 |
| Adriamycin | S. TA 98 |
| Methylglyoxal | S. TA 100 |

The respective mixtures were shaken for 20 minutes at 37°C. After adding 2.5 ml of a soft agar solution, the mixtures were spread on minimum glucose agar plates which had been supplemented with 0.1 μM/plate of histidine for causing several meioses of the cells. After incubation at 37°C for 48 hours, the number of colonies on the plates was counted as revertants.

(II) Results

(i) Inactivation of the mutagenicity of MNNG by calcium Pantothenate

Table 7

Efficacy of Calcium Pantothenate against MNNG

| Calcium pantothenate (μM/plate) | No. of revertants/ plate | (%)* |
|---|---|---|
| 0 | 220 | 100 |
| 0.005 | 196 | 89 |
| 0.05 | 212 | 96 |
| 0.5 | 115 | 52 |
| 5 | 47 | 21 |

* The number of revertants per plate in the absence of calcium pantothenate was taken as 100%.

MNNG: 1 μg (6.8 nM)/plate

Calcium pantothenate inactivated 48% of the mutagenicity of MNNG when 73 mols of the former was added to 1 mol of the latter.

(ii) Inactivating the mutagenicity of MNU

Table 8

Efficacy of Calcium Pantothenate against MNU

| Calcium pantothenate (μM/plate) | No. of revertants/ plate | (%)* |
|---|---|---|
| 0 | 1135 | 100 |
| 0.005 | 1087 | 96 |
| 0.05 | 979 | 86 |
| 0.5 | 371 | 33 |
| 5.0 | 2 | 0 |

* The number of revertants per plate in the absence of calcium pantothenate was taken as 100%.

MNU: 87.5 μg (0.85μM)/plate

Calcium pantothenate inactivated completely the mutagenicity of MNU when 6 mols of the former was added to 1 mol of the latter.

(iii) Inactivating the mutagenicity of 4NQO

Table 9

Efficacy of Calcium Pantothenate against 4NQO

| Calcium pantothenate (μM/plate) | No. of revertants/ plate | (%)* |
|---|---|---|
| 0 | 1679 | 100 |
| 0.005 | 1586 | 94 |
| 0.05 | 1846 | 110 |
| 0.5 | 1399 | 83 |
| 5.0 | 680 | 41 |

* The number of revertants per plate in the absence of calcium pantothenate was taken as 100%.

4NQO: 0.125 μg (0.66nM)/plate

Calcium pantothenate inactivated 17% of the mutagenicity of 4NQO when 760 mols of the former was added to 1 mol of the latter.

(iv) Inactivation of the mutagenicity of AF-2 by sodium pantothenate

Table 10

Efficacy of Sodium Pantothenate against AF-2

| Sodium pantothenate (μM/plate) | No. of revertants/ plate | (%)* |
|---|---|---|
| 0 | 1048 | 100 |
| 0.005 | 1082 | 103 |
| 0.05 | 1136 | 108 |
| 0.5 | 894 | 85 |
| 5.0 | 439 | 42 |

* The number of revertants per plate in the absence of sodium pantothenate was taken as 100%.

AF-2: 0.02 μg (0.8 nM)/plate

Sodium pantothenate inactivated 58% of the mutagenicity of AF-2 when 6250 mols of the former was added to 1 mol of the latter.

(v) Inactivating the mutagenicity of adriamycin

Table 11

Efficacy of sodium pantothenate against adriamycin

| Sodium pantothenate (μM/plate) | No. of revertants/ plate | (%)* |
|---|---|---|
| 0 | 872 | 100 |
| 0.005 | 964 | 111 |
| 0.05 | 664 | 76 |
| 0.5 | 361 | 41 |
| 5.0 | 278 | 32 |

* The number of revertants per plate in the absence of sodium pantothenate was taken as 100%.

Adriamycin: 0.125 μg (0.22 nM)/plate

Sodium pantothenate inactivated 59% of the mutagenicity of adriamycin when 2273 mols of the former was added to 1 mol of the latter.

(vi) Inactivating the mutagenicity of methylglyoxal

Table 12

Efficacy of Sodium Pantothenate against Methylglyoxal

| Sodium pantothenate (μM/plate) | No. of revertants/ plate | (%)* |
|---|---|---|
| 0 | 2456 | 100 |
| 0.005 | 2457 | 100 |
| 0.05 | 2333 | 95 |
| 0.5 | 1606 | 65 |
| 5.0 | 569 | 23 |

* The number of revertants per plate in the absence of sodium pantothenate was taken as 100%.

Methylglyoxal: 30 μg (0.28 μM)/plate

The above data shows that 18 mols of sodium pantothenate added to 1 mol of methylglyoxal could inactivate 77% of the mutagenicity of the latter.

Claims:

1. An antimutagenic agent having as its effective ingredient a compound selected from the group consisting of coenzyme A, pyridoxal-5'-phosphoric acid, and pantothenic acid or an alkali metal or alkaline earth metal salt thereof.

2. An antimutagenic agent according to Claim 1 wherein the effective ingredient is coenzyme A.

3. An antimutagenic agent according to Claim 1 wherein the effective ingredient is pyridoxal-5'-phosphoric acid.

4. An antimutagenic agent according to Claim 1 wherein the effective ingredient is pantothenic acid or an alkali metal or alkaline earth metal salt thereof.